# EUROPEAN PATENT APPLICATION

(11) **EP 4 685 195 A1**
(43) Date of publication of application: **28.01.2026**
(21) Application number: 24774825.4
(22) Date of filing: 14.03.2024
(51) Int. Cl.: C08L 101/00, C07C 271/12, C08K 5/24

(54) **DEGRADABLE CROSSLINKING AGENT**

(30) Priority: 20.03.2023 JP 2023044477
(71) Applicant: NAGASE CHEMTEX CORPORATION, Osaka-shi, Osaka 550-8668 (JP)
(72) Inventor: KURUSHIMA, Yasunori, Tatsuno-shi, Hyogo 679-4124 (JP)
(74) Representative: Klöckner, Christoph
(86) International application number: PCT/JP2024/010025
(87) International publication number: WO 2024/195687

(57) **Abstract**

The present invention provides a degradable cross-linking agent that is highly soluble in solvents. The present invention relates to a degradable cross-linking agent including a compound represented by the following formula (1), wherein - n ≥ 0, k ≥ 0, m ≥ 0, p1 ≥ 1, p2 ≥ 1, and p3 ≥ 1; - R¹, R², and R³ each independently represent a single bond or a C1-C500 hydrocarbon optionally containing a substituent or a heteroatom; - Q¹, Q², and Q³ each independently represent at least one reactive functional group selected from the group consisting of a hydroxy group, an amino group, a thiol group, a hydrazide group, a carboxylic acid group, an acid anhydride group, a vinyl group, an allyl group, an acrylate group, a methacrylate group, a crotonate group, an isoprenyl group, an acrylamide group, a methacrylamide group, a crotonamide group, an epoxy group, an oxetane group, an oxazoline group, an isocyanate group, a carbodiimide group, a methylol group, a silanol group, a hydroxysilyl group, and an alkoxysilyl group; - X^{1a}, X^{1b}, X^{2a}, X^{2b}, X^{3a}, X^{3b}, X^{4a}, and X^{4b} each independently represent an oxygen atom, a sulfur atom, a single bond, a C1-C20 hydrocarbon group optionally containing a substituent, or -NB- where B represents a hydrogen atom or a hydrocarbon group, and at least one of X^{1a}, X^{1b}, X^{2a}, X^{2b}, X^{3a}, X^{3b}, X^{4a}, and X^{4b} is an oxygen atom, a sulfur atom, or -NB- where B represents a hydrogen atom or a hydrocarbon group; - A¹ to A⁸ each represent a carbonyl group or a single bond, at least one of A¹ and A² is a carbonyl group, at least one of A³ and A⁴ is a carbonyl group, at least one of A⁵ and A⁶ is a carbonyl group, and at least one of A⁷ and A⁸ is a carbonyl group; and - each Z independently represents a divalent or higher-valent group containing a siloxane structure or a C l-C500 hydrocarbon group optionally containing a heteroatom.

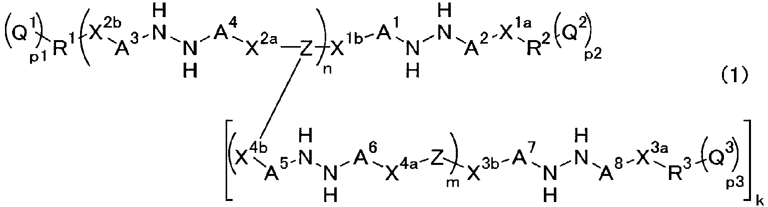

## Description

### TECHNICAL FIELD

The present invention relates to a degradable cross-linking agent.

### BACKGROUND ART

Plastic products are utilized across diverse fields, including daily necessities, automobiles, and electronic devices, owing to their formability, durability, and lightweight properties. However, they present the problem of poor degradability after disposal. In recent years, degradable polymers, which are easily degradable, have been developed for global environmental protection.

Polymers having a diacylhydrazine structure are disclosed as examples of degradable polymers (see, Patent Literatures 1, 2, and 3). These polymers are stable in the air due to the presence of the diacylhydrazine structure, while they are rapidly degraded when reacted with oxidizing agents such as sodium hypochlorite.

### CITATION LIST

### - Patent Literature

Patent Literature 1: JP 2011-236381 A
Patent Literature 2: WO 2021/131003
Patent Literature 3: JP 2011-052075 A

### SUMMARY OF INVENTION

### - Technical Problem

The diacylhydrazine compounds used as raw materials for the polymers described in Patent Literatures 1 and 2 and the diacylhydrazine-containing polymers described in Patent Literature 3 are poorly soluble in solvents, which significantly limits the formulation options when combined with materials such as resins or polymerization initiators. Moreover, even those with relatively high solvent solubility are only soluble in high-boiling-point aprotic polar solvents or strong acids. Accordingly, in applications such as paints, which involve a process of drying and removing a solvent, they pose significant environmental burdens and are difficult to use. The present invention aims to provide a degradable cross-linking agent that is highly soluble in solvents.

### - Solution to Problem

The present inventor has found that a hydrazine derivative containing a specific structure can exhibit enhanced solvent solubility. Based on this finding, the present invention has been completed.

Specifically, Embodiment 1 of the present disclosure relates to a degradable cross-linking agent including a compound represented by the following formula (1): 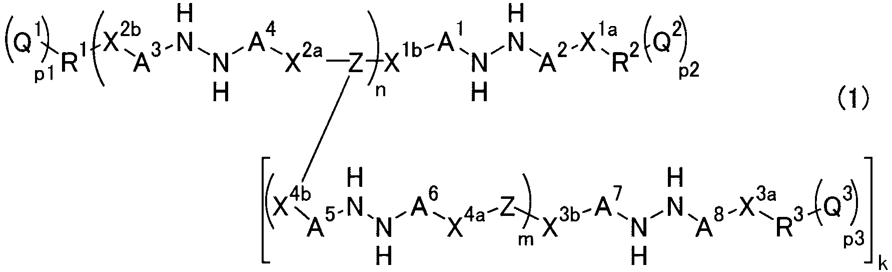 wherein
- n ≥ 0, k ≥ 0, m ≥ 0, p1 ≥ 1, p2 ≥ 1, and p3 ≥ 1;
- R¹, R², and R³ each independently represent a single bond or a C1-C500 hydrocarbon optionally containing a substituent or a heteroatom;
- Q¹, Q², and Q³ each independently represent at least one reactive functional group selected from the group consisting of a hydroxy group, an amino group, a thiol group, a hydrazide group, a carboxylic acid group, an acid anhydride group, a vinyl group, an allyl group, an acrylate group, a methacrylate group, a crotonate group, an isoprenyl group, an acrylamide group, a methacrylamide group, a crotonamide group, an epoxy group, an oxetane group, an oxazoline group, an isocyanate group, a carbodiimide group, a methylol group, a silanol group, a hydroxysilyl group, and an alkoxysilyl group;
- X^{1a}, X^{1b}, X^{2a}, X^{2b}, X^{3a}, X^{3b}, X^{4a}, and X^{4b} each independently represent an oxygen atom, a sulfur atom, a single bond, a C1-C20 hydrocarbon group optionally containing a substituent, or -NB- where B represents a hydrogen atom or a hydrocarbon group, and at least one of X^{1a}, X^{1b}, X^{2a}, X^{2b}, X^{3a}, X^{3b}, X^{4a}, and X^{4b} is an oxygen atom, a sulfur atom, or -NB- where B represents a hydrogen atom or a hydrocarbon group;
- A¹ to A⁸ each represent a carbonyl group or a single bond, at least one of A¹ and A² is a carbonyl group, at least one of A³ and A⁴ is a carbonyl group, at least one of A⁵ and A⁶ is a carbonyl group, and at least one of A⁷ and A⁸ is a carbonyl group; and
- each Z independently represents a divalent or higher-valent group containing a siloxane structure or a C1-C500 hydrocarbon group optionally containing a heteroatom.

Embodiment 2 of the present disclosure is the degradable cross-linking agent according to Embodiment 1,
wherein p1 ≥ 2, p2 ≥ 2, or p3 ≥ 2, and
the plurality of reactive functional groups as Q¹ are different from each other, or the plurality of reactive functional groups as Q² are different from each other, or the plurality of reactive functional groups as Q³ are different from each other.

Embodiment 3 of the present disclosure is the degradable cross-linking agent according to Embodiment 1 or 2,
wherein p1 = 1, p2 = 1, or p3 = 1.

Embodiment 4 of the present disclosure is the degradable cross-linking agent according to Embodiment 1 or 2, which has at least one degradable moiety represented by the following formula (2):

-X⁵-C¹-NH-NH-C²-X⁶- (2)

wherein X⁵ and X⁶ are different from each other and each represent an oxygen atom, a sulfur atom, a single bond, a C1-C20 hydrocarbon group optionally containing a substituent, or -NB- where B represents a hydrogen atom or a hydrocarbon group; and C¹ and C² each represent a carbonyl group or a single bond, and at least one of C¹ and C² is a carbonyl group.

Embodiment 5 of the present disclosure is the degradable cross-linking agent according to Embodiment 1 or 2,
wherein X^{1a} or X^{1b} is an oxygen atom.

Embodiment 6 of the present disclosure is the degradable cross-linking agent according to Embodiment 1 or 2,
wherein n ≥ 1.

Embodiment 7 of the present disclosure is the degradable cross-linking agent according to Embodiment 1 or 2,
wherein n ≥ 1,
at least one of X^{1a}, X^{1b}, X^{2a}, X^{2b}, X^{3a}, X^{3b}, X^{4a}, and X^{4b} is an oxygen atom,
at least one of X^{1a}, X^{1b}, X^{2a}, X^{2b}, X^{3a}, X^{3b}, X^{4a}, and X^{4b} is a single bond or a C1-C20 hydrocarbon group optionally containing a substituent, and
at least one of X^{1a}, X^{1b}, X^{2a}, X^{2b}, X^{3a}, X^{3b}, X^{4a}, and X^{4b} is -NB- where B represents a hydrogen atom or a hydrocarbon group.

Embodiment 8 of the present disclosure is the degradable cross-linking agent according to Embodiment 1 or 2, which has a molecular weight of 2000 or less.

Embodiment 9 of the present disclosure is the degradable cross-linking agent according to Embodiment 1 or 2,
wherein k ≥ 1.

Embodiment 10 of the present disclosure relates to a composition containing:
the degradable cross-linking agent according to Embodiment 1 or 2, and
at least one selected from the group consisting of a curable resin, a polymerization initiator, and a solvent.

Embodiment 11 of the present disclosure is the composition according to Embodiment 10, which contains substantially no solvent with a boiling point of higher than 150°C.

Embodiment 12 of the present disclosure relates to a degradable cross-linked product formed from the composition according to Embodiment 10.

Embodiment 13 of the present disclosure is the degradable cross-linked product according to Embodiment 12, which is degradable with an oxidizing agent.

Embodiment 14 of the present disclosure relates to a method for degrading a degradable cross-linked product, including
bringing the degradable cross-linked product according to Embodiment 12 into contact with an aqueous solution containing an oxidizing agent at 100°C or lower.

### - Advantageous Effects of Invention

The degradable cross-linking agent of the present invention has excellent solvent solubility.

### DESCRIPTION OF EMBODIMENTS

### <<Degradable cross-linking agent>>

The degradable cross-linking agent of the present invention includes a compound represented by the following formula (1): 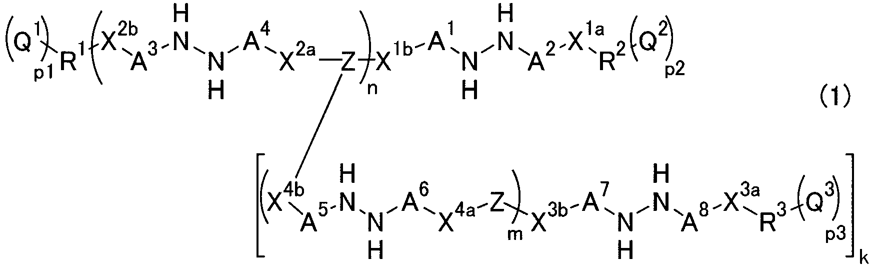 wherein
- n ≥ 0, k ≥ 0, m ≥ 0, p1 ≥ 1, p2 ≥ 1, and p3 ≥ 1;
- R¹, R², and R³ each independently represent a single bond or a C1-C500 hydrocarbon optionally containing a substituent or a heteroatom;
- Q¹, Q², and Q³ each independently represent at least one reactive functional group selected from the group consisting of a hydroxy group, an amino group, a thiol group, a hydrazide group, a carboxylic acid group, an acid anhydride group, a vinyl group, an allyl group, an acrylate group, a methacrylate group, a crotonate group, an isoprenyl group, an acrylamide group, a methacrylamide group, a crotonamide group, an epoxy group, an oxetane group, an oxazoline group, an isocyanate group, a carbodiimide group, a methylol group, a silanol group, a hydroxysilyl group, and an alkoxysilyl group;
- X^{1a}, X^{1b}, X^{2a}, X^{2b}, X^{3a}, X^{3b}, X^{4a}, and X^{4b} each independently represent an oxygen atom, a sulfur atom, a single bond, a C1-C20 hydrocarbon group optionally containing a substituent, or -NB- where B represents a hydrogen atom or a hydrocarbon group, and at least one of X^{1a}, X^{1b}, X^{2a}, X^{2b}, X^{3a}, X^{3b}, X^{4a}, and X^{4b} is an oxygen atom, a sulfur atom, or -NB- where B represents a hydrogen atom or a hydrocarbon group;
- A¹ to A⁸ each represent a carbonyl group or a single bond, at least one of A¹ and A² is a carbonyl group, at least one of A³ and A⁴ is a carbonyl group, at least one of A⁵ and A⁶ is a carbonyl group, and at least one of A⁷ and A⁸ is a carbonyl group; and
- each Z independently represents a divalent or higher-valent group containing a siloxane structure or a C1-C500 hydrocarbon group optionally containing a heteroatom.

The degradable cross-linking agent of the present invention is characterized in that at least one of the plurality of X groups in formula (1) is an oxygen atom or -NB- where B represents a hydrogen atom or a hydrocarbon group. Thus, the degradable cross-linking agent has high solvent solubility.

It should be noted that although the term "cross-linking agent" may narrowly refer to a chemical substance capable of forming a chemical bond between polymers or within a polymer, the term as used herein refers not only to a chemical substance capable of forming a chemical bond between polymers or within a polymer but also to a chemical substance capable of forming a chemical bond between the molecules of the chemical substance itself.

### <n, m, and k in formula (1)>

In formula (1), n and m both represent 0 or more and are each independently preferably 1 to 50, more preferably 1 to 5. When n is 1 or more, the degradable cross-linking agent can exhibit enhanced degradability due to the presence of two or more hydrazine structures (-A-NH-NH-A-). Additionally, the solvent solubility can be finely tuned by selecting not only X^{1a} and X^{1b}, but also X^{2a} and X^{2b}. The upper limit of n or m is not limited, but, if it exceeds 50, the solvent solubility tends to decrease.

In formula (1), k represents 0 or more, preferably 1 or more, more preferably 2 or more. When k is 1 or more, three-dimensional cross-linking can be performed with the degradable cross-linking agent of the present invention. The upper limit of k is not limited and may be 200 or less. If k is more than 200, the solvent solubility tends to decrease.

### <p1, p2, and p3 in formula (1)>

In formula (1), p1, p2, and p3 each represent 1 or more and are each independently preferably 1 to 4, more preferably 1 to 2. When p1 ≥ 2, p2 ≥ 2, or p3 ≥ 2, the degradable cross-linking agent of the present invention can facilitate three-dimensional cross-linking, improving the strength and reliability of the cross-linked product. When p1 ≥ 2, p2 ≥ 2, or p3 ≥ 2, and the plurality of reactive functional groups as Q¹ are different from each other, or the plurality of reactive functional groups as Q² are different from each other, or the plurality of reactive functional groups as Q³ are different from each other, the cross-linking agent allows for flexible selection of various curable resins or cross-linking processes. Moreover, when only a specific reactive functional group is cross-linked, the reactive functional groups not involved in the cross-linking reaction can contribute to improving other physical properties such as adhesiveness or solubility. When p1 = 1, p2 = 1, or p3 = 1, the solvent solubility and the degradation rate can be further enhanced.

### <R¹, R², and R³ in formula (1)>

In formula (1), R¹, R², and R³ each independently represent a single bond or a C1-C500 hydrocarbon group optionally containing a substituent or a heteroatom. When R¹, R², or R³ is the hydrocarbon group, the number of carbon atoms thereof is 1 to 500. In view of the degradation rate, the number of carbon atoms is preferably 1 to 100, more preferably 1 to 10. Examples of the substituent include an alkoxy group, a phenoxy group, a halogen atom, a tertiary amino group, and a sulfo group.

The hydrocarbon group has a saturated or unsaturated hydrocarbon group as its main backbone, which may have a branched or linear structure. Moreover, the backbone formed of the hydrocarbon group may have a cyclic structure. The hydrocarbon group may contain a heteroatom such as N, S, or O. The heteroatom may be present on the main chain or a side chain of the hydrocarbon group. Examples of the heteroatom-containing structure include an ether bond, a urethane bond, a urea bond, - NH- in which the hydrogen atom may be substituted, a silicone bond, an ester bond, a thioether bond, and a carbonate bond. The methylene groups other than those at the terminals in the hydrocarbon group may be replaced by one to four heteroatoms selected from N, S, O, and the like, or by arylene or heteroarylene groups. The hydrogen atoms in the hydrocarbon group may be replaced by cyano, nitro, halogen, or phenyl. However, the structure of R¹, R², or R³ preferably contains no disulfide bond because disulfide bonds may be converted to sulfonic acid by an oxidizing agent.

Examples of the hydrocarbon group include linear hydrocarbons such as methylene, ethylene, propylene, butylene, pentylene, hexylene, heptylene, octylene, and nonylene; branched hydrocarbons such as isopropylene, isobutylene, 2,2-dimethylpropylene, and 2-ethyl-2-methylpropylene; cyclic hydrocarbons such as cyclohexylene, cyclopentylene, norbomenylene, phenylene, and naphthylene; and PEG chains, as well as trivalent or tetravalent groups corresponding to the foregoing groups.

### <Q¹, Q², and Q³ in formula (1)>

In formula (1), Q¹, Q², and Q³ each independently represent at least one reactive functional group selected from the group consisting of a hydroxy group, an amino group, a thiol group, a hydrazide group, a carboxylic acid group, an acid anhydride group, a vinyl group, an allyl group, an acrylate group, a methacrylate group, a crotonate group, an isoprenyl group, an acrylamide group, a methacrylamide group, a crotonamide group, an epoxy group, an oxetane group, an oxazoline group, an isocyanate group, a carbodiimide group, a methylol group, a silanol group, a hydroxysilyl group, and an alkoxysilyl group. The type of reactive functional group may be selected optimally depending on the application or process.

### <X in formula (1)>

In formula (1), X^{1a}, X^{1b}, X^{2a}, X^{2b}, X^{3a}, X^{3b}, X^{4a}, and X^{4b} each independently represent an oxygen atom, a sulfur atom, a single bond, a C1-C20 hydrocarbon group optionally containing a substituent, or -NB- where B represents a hydrogen atom or a hydrocarbon group. Hereinbelow, X^{1a}, X^{1b}, X^{2a}, X^{2b}, X^{3a}, X^{3b}, X^{4a}, and X^{4b} are collectively referred to as X groups.

When X is the hydrocarbon group, the number of carbon atoms thereof is 1 to 20. In view of ease of handling and facilitation of the cross-linking reaction, the number of carbon atoms is preferably 1 to 10, more preferably 1 to 6.

The hydrocarbon group has a saturated or unsaturated hydrocarbon group as its main backbone. The hydrocarbon group may have a branched or linear structure. Moreover, the backbone formed of the hydrocarbon group may have a cyclic structure.

The hydrocarbon group may also contain a heteroatom such as N, S, or O in the molecular chain. The methylene groups other than those at the terminals in the hydrocarbon group may be replaced by one to four heteroatoms selected from N, S, and O, or by arylene or heteroarylene groups. The hydrogen atoms in the hydrocarbon group may be replaced by cyano, nitro, halogen, or phenyl.

Specific examples of the hydrocarbon group include linear hydrocarbons such as methylene, ethylene, propylene, butylene, pentylene, hexylene, heptylene, octylene, and nonylene; branched hydrocarbons such as 2,2-dimethylpropylene and 2-ethyl-2-methylpropylene; and cyclic hydrocarbons such as 1,4-cyclohexylene, cyclopentylene, norbomenylene, phenylene, and naphthylene. Methylene or phenylene is preferred among these.

When X is -NB-, B represents a hydrogen atom or a hydrocarbon group. When B is a hydrocarbon group, the number of carbon atoms thereof is preferably 1 to 10, more preferably 1 to 5, in view of the degradation rate. The hydrocarbon group B has a saturated or unsaturated hydrocarbon group as its main backbone, which may have a branched or linear structure. Moreover, the backbone formed of the hydrocarbon group may have a cyclic structure.

The hydrocarbon group B may also contain a heteroatom such as N, S, or O in the molecular chain. The methylene groups other than those at the terminals in the hydrocarbon group may be replaced by one to four heteroatoms selected from N, S, and O, or by arylene or heteroarylene groups. The hydrogen atoms in the hydrocarbon group may be replaced by cyano, nitro, halogen, or phenyl.

Specific examples of the hydrocarbon group B include linear hydrocarbons such as methylene, ethylene, propylene, butylene, pentylene, hexylene, heptylene, octylene, and nonylene; branched hydrocarbons such as 2,2-dimethylpropylene and 2-ethyl-2-methylpropylene; cyclic hydrocarbons such as 1,4-cyclohexylene, cyclopentylene, norbornenylene, phenylene, and naphthylene; and PEG chains, as well as trivalent or tetravalent groups corresponding to the foregoing groups. Linear hydrocarbons are preferred among these.

When at least one X in formula (1) is an oxygen atom, a sulfur atom, or -NB- where B represents a hydrogen atom or a hydrocarbon group, the general solvent solubility is enhanced. Moreover, the solvent solubility can be finely tuned by selecting the plurality of X groups. To provide excellent solvent solubility, any one of X^{1a}, X^{1b}, X^{2a}, X^{2b}, X^{3a}, X^{3b}, X^{4a}, and X^{4b} is preferably an oxygen atom, and X^{1a} or X^{1b} is more preferably a hydrogen atom.

Still more preferably, in formula (1), n ≥ 1, at least one of X^{1a}, X^{1b}, X^{2a}, X^{2b}, X^{3a}, X^{3b}, x^{4a}, and X^{4b} is an oxygen atom, at least one of X^{1a}, X^{1b}, X^{2a}, X^{2b}, X^{3a}, X^{3b}, x^{4a}, and X^{4b} is a single bond or a C1-C20 hydrocarbon group optionally containing a substituent, and at least one of X^{1a}, X^{1b}, X^{2a}, X^{2b}, X^{3a}, X^{3b}, X^{4a}, and X^{4b} is -NB- where B represents a hydrogen atom or a hydrocarbon group. Also preferably, in formula (1), n ≥ 1, and X^{1a}, X^{1b}, X^{2a}, X^{2b}, X^{3a}, X^{3b}, X^{4a}, and X^{4b} include at least three selected from the group consisting of an oxygen atom, a sulfur atom, a single bond, or a C1-C20 hydrocarbon group optionally containing a substituent, and -NB- where B represents a hydrogen atom or a hydrocarbon group. When three or more X groups differ from each other in structure as described above, the solvent solubility can be enhanced.

The degradable cross-linking agent preferably has different structures at both ends of the hydrazine structure (-A-NH-NH-A-). Such a structure is represented by the following formula (2):

-X⁵-C¹-NH-NH-C²-X⁶- (2)

wherein X⁵ and X⁶ are different from each other and each represent an oxygen atom, a sulfur atom, a single bond, a C1-C20 hydrocarbon group optionally containing a substituent, or -NB- where B represents a hydrogen atom or a hydrocarbon group; and C¹ and C² each represent a carbonyl group or a single bond, and at least one of C¹ and C² is a carbonyl group. The combination of X⁵ and X⁶ is preferably a combination of an oxygen atom and a single bond or a C1-C20 hydrocarbon group optionally containing a substituent or a combination of an oxygen atom and -NB- where B represents a hydrogen atom or a hydrocarbon group.

Moreover, the combinations of X groups at both ends of each hydrazine structure (-A-NH-NH-A-), specifically, the combination of X^{1a} and X^{1b}, the combination of X^{2a} and X^{2b}, the combination of X^{3a} and X^{3b}, and the combination of X^{4a} and X^{4b} may be the same as or different from each other. The solvent solubility can be finely tuned through the design of these combinations. In exemplary combinations differing from each other, X^{1a} is an oxygen atom, X^{1b} is a single bond or a C1-C20 hydrocarbon group optionally containing a substituent, X^{2a} is an oxygen atom, and X^{2b} is -NB- where B represents a hydrogen atom or a hydrocarbon group.

### <A¹ to A⁸ in formula (1)>

In formula (1), A¹ to A⁸ each represent a carbonyl group or a single bond, at least one of A¹ and A² is a carbonyl group, at least one of A³ and A⁴ is a carbonyl group, at least one of A⁵ and A⁶ is a carbonyl group, and at least one of A⁷ and A⁸ is a carbonyl group.

When one of the two A groups present at both ends of -NH-NH- is a carbonyl group, the rate of degradation with an oxidizing agent tends to be enhanced. When both A groups are carbonyl groups, the degradation product tends to dissolve easily in a solution containing an oxidizing agent.

### <Z in formula (1)>

In formula (1), the plurality of Z groups each independently represent a divalent or higher-valent group containing a siloxane structure or a C1-C500 hydrocarbon group optionally containing a heteroatom.

When Z is a divalent or higher-valent group containing a siloxane structure, the group containing a siloxane structure has a -Si-O-bond as its main backbone. The main backbone may have a linear, branched, or cyclic structure. The number of silicon atoms in the group containing a siloxane structure is preferably 2 to 400, more preferably 4 to 200, still more preferably 8 to 150.

In the siloxane structure, the hydrogen atom bound to a silicon atom may be substituted. Specific examples of the substituent include an alkyl group, an alkoxy group, a phenoxy group, a halogen atom, an amino group, a sulfo group, a cyano group, and a nitro group.

Specific examples of the group containing a siloxane structure include dimethyl silicone, diethyl silicone, ethyl methyl silicone, and polymethylsilsesquioxane, as well as modified silicones obtained by modifying a hydrocarbon group at the terminal and/or on a side chain of any of the foregoing compounds to incorporate a heteroatom such as N, S, O, or P.

When Z is a C1-C500 hydrocarbon group optionally containing a heteroatom, the number of carbon atoms thereof is more preferably 1 to 200, still more preferably 2 to 30, in view of the degradation rate and crosslink density. The hydrocarbon group has a saturated or unsaturated hydrocarbon group as its main backbone, which may have a branched or linear structure. Moreover, the backbone formed of the hydrocarbon group may have a cyclic structure. The hydrocarbon group may contain a heteroatom such as N, S, or O. The heteroatom may be present on the main chain or a side chain of the hydrocarbon group. Examples of the heteroatom-containing structure include an ether bond, a urethane bond, a urea bond, -NH- in which the hydrogen atom may be substituted, a silicone bond, an ester bond, a thioether bond, and a carbonate bond. The methylene groups other than those at the terminals in the hydrocarbon group may be replaced by one to four heteroatoms selected from N, S, O, and the like, or by arylene or heteroarylene groups. The hydrogen atoms in the hydrocarbon group may be replaced by hydroxy, cyano, amino, nitro, halogen, or phenyl. However, the structure of Z preferably contains no disulfide bond because disulfide bonds may be converted to sulfonic acid by an oxidizing agent.

Examples of the hydrocarbon group include linear hydrocarbons such as methylene, ethylene, propylene, butylene, pentylene, hexylene, heptylene, octylene, and nonylene; branched hydrocarbons such as isopropylene, isobutylene, 2,2-dimethylpropylene, and 2-ethyl-2-methylpropylene; cyclic hydrocarbons such as cyclohexylene, cyclopentylene, norbornenylene, phenylene, naphthylene, and pyridylene; and PEG chains, as well as trivalent or tetravalent groups corresponding to the foregoing groups.

### <Molecular weight>

The molecular weight of the degradable cross-linking agent is not limited, but is preferably 20000 or less, more preferably 400 to 2000, still more preferably 700 to 2000. When the molecular weight is within the above range, the balance between degradability and crosslink density tends to improve. If the molecular weight is more than 20000, the solvent solubility tends to decrease.

### <Method for synthesizing degradable cross-linking agent>

The degradable cross-linking agent may be synthesized by any method. Examples of such methods include a reaction of a hydrazide compound, a semicarbazide compound, or a carbazate compound with a carbonate compound, an isocyanate compound, an acid anhydride, an acid halide, or a cyclic ester compound; a reaction of hydrazine with a carboxylic acid ester; a reaction of hydrazine with a carbonate compound; and a reaction of hydrazine with an isocyanate compound.

Examples of hydrazide compounds that can be used in the synthetic method include lactic acid hydrazide, methacrylic acid hydrazide, sebacic acid dihydrazide, adipic acid dihydrazide, phthalic acid dihydrazide, salicylic acid dihydrazide, and trimellitic acid trihydrazide. Examples of carbonate compounds include allyl N-succinimidyl carbonate and C,C'-(oxydi-2,1-ethanediyl)bis-N-succinimidyl carbonate. Examples of isocyanate compounds include 2-isocyanatoethyl methacrylate, hexamethylene diisocyanate, toluene diisocyanate, and polymethylene polyphenyl polyisocyanate. Examples of semicarbazide compounds include N-allylhydrazinecarboxamide, N,N'-1,6-hexanediylbis[hydrazinecarboxamide], 4,4'-isophoronebis(semicarbazide), and 4,4'-(1,3-phenylenebismethylene)bis(semicarbazide). Examples of carbazate compounds include allyl carbazate and C,C'-(oxydi-2,1-ethanediyl)biscarbazate. Examples of acid anhydrides include methacrylic anhydride, succinic anhydride, and pyromellitic dianhydride. Examples of acid halides include acryloyl chloride, sebacoyl chloride, adipoyl chloride, phthaloyl chloride, salicyloyl chloride, and trimesoyl chloride. Examples of cyclic ester compounds include propiolactone, butyrolactone, and valerolactone. Examples of carboxylic acid esters include ethyl lactate, methylparaben, monomethyl succinate, diethyl adipate, and trimethyl trimellitate.

### <Composition>

The composition according to the present invention contains the above-described degradable cross-linking agent and at least one selected from the group consisting of a curable resin, a polymerization initiator, and a solvent. The degradable cross-linking agent content in the composition is preferably 0.1 to 99% by weight, more preferably 1 to 50% by weight.

### <Curable resin>

The curable resin may be any curable resin having a structure that can react with a reactive functional group in the degradable cross-linking agent for cross-linking. Examples of the curable resin include acrylic resins, phenolic resins, epoxy resins, melamine resins, urea resins, unsaturated polyester resins, alkyd resins, silicone resins, isocyanate compounds, and polyimides. The curable resin may be a thermosetting resin or a photocurable resin. The curable resin content in the composition is preferably 0.1 to 95% by weight, more preferably 1 to 50% by weight.

### <Polymerization initiator>

The polymerization initiator may be any compound that can catalyze the polymerization of the curable resin and may be either a thermal polymerization initiator or a photopolymerization initiator. Examples of the polymerization initiator include radical generators such as alkylphenone compounds, benzoin compounds, benzophenone compounds, oxime ester compounds, and phosphine compounds; base generators such as oxime ester compounds, ammonium compounds, benzoin compounds, dimethoxybenzyl urethane compounds, and o-nitrobenzyl urethane compounds; acid generators such as onium salts, halogen-containing compounds, diazomethane compounds, sulfone compounds, and sulfonate compounds; tin compounds such as dibutyltin dilaurate and dibutyltin diacetate; bismuth compounds such as bismuth 2-ethylhexanoate; titanium compounds such as tetraoctyl titanate and titanium ethyl acetoacetate; zirconium compounds such as zirconium monoacetylacetate and zirconium tetraacetylacetate; amines such as triethylenediamine and 1,4-diazabicyclo[2,2,2]octane (DABCO); platinum compounds such as chloroplatinic acid and alkenylsiloxane platinum complexes; iron complexes; cobalt complexes; and nickel complexes. The polymerization initiator content in the composition is preferably 0.1 to 10 parts by weight, more preferably 1 to 5 parts by weight, per 100 parts by weight of the curable resin content.

### <Solvent>

Examples of the solvent include water and organic solvents. Examples of the organic solvents include solvents containing carbon atoms, such as ether solvents, amide solvents, hydrocarbon solvents, alcohol solvents, ester solvents, aldehyde solvents, ketone solvents, and solvents containing carbon atoms and heteroatoms.

Examples of the ether solvents include propylene glycol monomethyl ether, anisole, 4-methylanisole, diisopropyl ether, diethyl ether, dibutyl ether, tetrahydrofuran, dimethoxyethane, cyclopentyl methyl ether, and tert-butyl methyl ether. Examples of the amide solvents include dimethylformamide, dimethylacetamide, and N-methylpyrrolidone. Examples of the hydrocarbon solvents include aliphatic hydrocarbon solvents such as pentane, hexane, heptane, octane, nonane, decane, undecane, dodecane, and cyclohexane, and aromatic hydrocarbon solvents such as benzene, toluene, xylene, mesitylene, ethylbenzene, tert-butylbenzene, trifluoromethylbenzene, nitrobenzene, and chlorobenzene. Examples of the alcohol solvents include ethanol, propanol, butanol, ethylene glycol, and propylene glycol monomethyl ether. Examples of the ester solvents include ethyl acetate, butyl acetate, and propylene glycol monomethyl ether acetate. Examples of the aldehyde solvents include formaldehyde and acetaldehyde. Examples of the ketone solvents include acetone, methyl ethyl ketone, and methyl isobutyl ketone. Examples of the solvents containing carbon atoms and heteroatoms include acetonitrile and dimethyl sulfoxide. The solvent content in the composition is preferably 1 to 99% by weight, more preferably 20 to 80% by weight.

The above-listed solvents may be used alone or in combination of two or more. Moreover, the solvent used preferably has a boiling point of 150°C or lower, more preferably 130°C or lower, still more preferably 110°C or lower. When the boiling point is within the above range, the solvent can be removed by heating for a relatively short time. The composition preferably contains substantially no solvent with a boiling point of higher than 150°C. The phrase "contains substantially no solvent with a boiling point of higher than 150°C" means that the content in the composition is less than 1% by weight.

### <Degradable cross-linked product>

The degradable cross-linked product according to the present invention can be obtained by curing a composition that contains the degradable cross-linking agent and at least one selected from the group consisting of a curable resin, a polymerization initiator, and a solvent. The degradable cross-linked product may be produced from the composition by any method that can promote the polymerization and cross-linking of the curable resin. A person skilled in the art can appropriately select the conditions for light irradiation, heating, or other processes depending on the types of the degradable cross-linking agent, the curable resin, and the polymerization initiator contained in the composition. For example, a three-dimensional formed body of the degradable cross-linked product can be produced by injection molding, compression molding, transfer molding, 3D printing, photolithography, or other methods

Moreover, a coating film including the degradable cross-linked product can be formed by applying the composition onto a substrate to form a coating film, and subjecting the coating film to light irradiation or heating. The material or form of the substrate is not limited and may be a resin, an inorganic material, paper, or fabric. Examples of the resin include polyesters such as polyethylene terephthalate, polyethylene naphthalate, polylactic acid, polyhydroxy butyric acid, and polybutylene succinate; polyolefins such as polyethylene, polypropylene, and polymethylpentene; cycloolefins, polystyrenes, polytetrafluoroethylene, PMMA, polyamides such as nylon 6 and nylon 66, polycarbonates, poly(vinyl acetate), poly(vinyl alcohol), polyimides, ABS resins, cellulose, cellulose acetate, fibroin, and keratin. Examples of the inorganic material include glass, metals such as Ni, Cu, Cr, Fe, and Si, and oxides or composite materials thereof.

Examples of the method for applying the composition onto the substrate include bar coating, spin coating, spray coating, dip coating, nozzle coating, gravure coating, reverse roll coating, die coating, air doctor coating, blade coating, rod coating, curtain coating, knife coating, transfer roll coating, squeeze coating, impregnation coating, kiss coating, calender coating, and extrusion coating.

The composition applied on the substrate can be cured under any condition. The conditions for curing by light irradiation may include a light irradiation dose of 100 to 2000 mJ/cm². Curing by heating may be performed preferably at a heating temperature of 40°C to 300°C, more preferably 80°C to 120°C. Moreover, the duration of heating is preferably 0.5 to 180 minutes, more preferably 0.5 to 10 minutes.

The thickness of the cured coating film is not limited, but is preferably 0.01 to 900 µm, more preferably 0.02 to 100 µm, still more preferably 0.05 to 10 µm. The coating film having a thickness within the above range can maintain the strength and adhesion to the substrate.

### <Method for degrading degradable cross-linked product>

The method for degrading a degradable cross-linked product according to the present invention includes bringing the degradable cross-linked product into contact with an aqueous solution containing an oxidizing agent at 100°C or lower.

### <Oxidizing agent>

The oxidizing agent may be any oxidizing agent other than molecular oxygen. Examples include sodium hypochlorite, potassium hypochlorite, calcium hypochlorite, ammonium hypochlorite, hydrogen peroxide, peracetic acid, m-chloroperoxybenzoic acid, peroxybenzoic acid, ammonium hypobromite, calcium hypobromite, potassium hypobromite, sodium hypobromite, and ozone. These may be used alone or in combination of two or more. In particular, a water-soluble salt such as sodium hypochlorite or sodium hypobromite or ozone water is preferred. The oxidizing agent is preferably reacted in the form of a degradation solution prepared by mixing the oxidizing agent with an alkali aqueous solution such as a sodium hydroxide aqueous solution or an organic solvent, a surfactant, etc. The oxidizing agent concentration in the aqueous solution is preferably 0.001 to 50% by weight, more preferably 0.01 to 3% by weight.

The conditions for bringing the degradable cross-linked product into contact with the aqueous solution containing the oxidizing agent include a temperature of 100°C or lower, preferably of 15°C to 50°C. The conditions preferably include a duration of 60 minutes or shorter, more preferably 10 minutes or shorter. If necessary, the cross-linked product may be shaken or stirred during the reaction with the oxidizing agent. Non-limiting specific examples of the method for bringing the degradable cross-linked product into contact with the aqueous solution containing the oxidizing agent include immersing the degradable cross-linked product in the aqueous solution containing the oxidizing agent, or spraying or adding dropwise the aqueous solution containing the oxidizing agent onto the degradable cross-linked product.

### <Degradation product>

When n ≥ 1 in the above formula (1), the degradable cross-linked product can be reacted with an oxidizing agent to generate a degradation product represented by the following formula:

X-Z(X)ₖ-X

wherein Z and k are each as defined in formula (1), and X is as defined for X^{1a}, X^{1b}, X^{2a}, X^{2b}, X^{3a}, X^{3b}, X^{4a}, and X^{4b} in formula (1). When X is a hydrocarbon group or a single bond, the degradation product forms a carboxylic acid group. When X is an oxygen atom, the degradation product forms an alcohol. When X is -NB- where B represents a hydrogen atom or a hydrocarbon group, the degradation product forms an amine. When X is a sulfur atom, the degradation product forms a thiol. When the plurality of X groups in the degradable cross-linking agent are different from each other, the degradation product may contain at least two selected from the group consisting of a carboxylic acid group, a hydroxy group, an amino group, and a thiol group.

The water solubility of the degradation product is preferably 30 g/L or more, more preferably 60 g/L or more. The water solubility is measured in water having a temperature of 20°C. When the degradation product to be generated has high water solubility, the cross-linked product shows an excellent degradation rate. This is presumably because the degradation product dissolves easily in an aqueous solution containing an oxidizing agent, which can prevent blockage of the reaction between the oxidizing agent and the cross-linking agent due to deposition of the degradation product, thereby promoting penetration of the aqueous oxidizing agent solution into the cross-linked product. However, the present invention is not limited to the above mechanism.

The degradation products may include a poorly water-soluble degradation product including a Z-containing carboxylic acid, a Z-containing alcohol, a Z-containing amine, or a Z-containing thiol and having low water solubility. Here, the term "poorly water-soluble degradation product" encompasses a substance having low water solubility as well as a substance that is not dissolved in water at all. Specific examples of the poorly water-soluble degradation product include terephthalic acid, 1,10-decanediol, 4,4'-methylenedianiline, and 1,2-ethanedithiol.

### <Application of degradable cross-linking agent>

Since the degradable cross-linking agent of the present invention has excellent solvent solubility, it can be used for cross-linking various resins. Examples of applications of the cross-linked product obtained using the degradable cross-linking agent of the present invention include adhesives, pressure-sensitive adhesives, water-absorbent resins, resins for 3D printing, photoresists, release agents, cell culture media, immobilization materials for affected areas, and imprint molded articles.

### EXAMPLES

The present invention is described below with reference to examples, but the present invention is not limited thereto. Hereinafter, the units "part(s)" and "%" refer to "part(s) by weight" and "% by weight", respectively, unless otherwise specified.

### (Comparative Example 1)

An amount of 1.4 g of pyridine, 10.3 g of acetonitrile, and 0.86 g of allylacetohydrazide were mixed in a 30 mL two-necked flask. A solution of 0.82 g of sebacoyl chloride in 6.2 g of acetonitrile was added dropwise to the mixture at 5°C or lower and then stirred at room temperature for three hours to obtain a white solid. The white solid was washed with 30 g of acetonitrile and dried in a vacuum dryer at 40°C to obtain a compound of formula (A) at a yield of 95%.

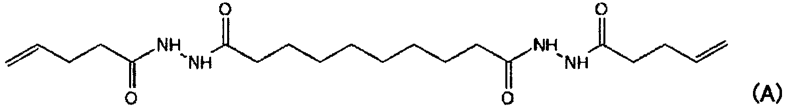

1H-NMR (DMSO, δ ppm) 1.25(8H, S, C8H16), 1.50(4H, t, C8H16), 2.09(4H, t, C8H16), 2.17 to 2.18(8H, m, C2H4), 4.96(2H, d, CH=CH2), 5.05(2H, d, CH=CH2), 5.76 to 5.87(2H, m, CH=CH2), 9.66(2H, S, NHNH), 9.69(2H, S, NHNH)
The solvent solubility of the obtained compound of formula (A) was evaluated. Table 1 shows the results. Upon adding a 3% sodium hypochlorite aqueous solution to the powdery compound of formula (A), foaming and degradation of the compound were observed.

An amount of 0.3 g of the compound of formula (A), 0.1 g of poly(methylhydrosiloxane), and 4.0 mg of a platinum catalyst (trade name: CAT-PL-50T, available from Shin-Etsu Chemical Co., Ltd.) were added to 40 g of dimethyl sulfoxide to obtain a thermosetting composition solution. The solution was applied onto a glass substrate to give a cured film thickness of 0.1 µm, and then dried and cured at 150°C for 10 minutes to obtain a degradable cross-linked product. The degradable cross-linked product was insoluble in water, but when it was immersed in a 3% sodium hypochlorite aqueous solution, foaming and removal of the product from the substrate were observed.

### (Example 1)

An amount of 15.7 g of dimethyl sulfoxide and 0.50 g of sebacic acid dihydrazide were mixed in a Schlenk flask at 80°C, and 0.46 g of allyl isocyanate was added dropwise to the mixture, followed by stirring for four hours. The resulting reaction liquid was returned to room temperature and then added dropwise to 150 g of acetone to obtain a white solid. The white solid was washed with acetone and dried in a vacuum dryer at room temperature to obtain a compound of formula (B) at a yield of 84%.

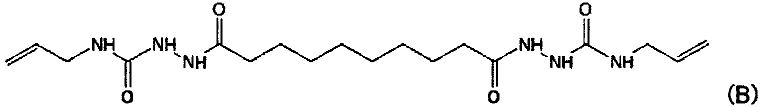

1H-NMR (DMSO, δ ppm) 1.24(8H, S, C8H16), 1.49(4H, t, C8H16), 2.08(4H, t, C8H16), 3.61 to 3.64(4H, m, CH2), 5.01(2H, dd, CH=CH2), 5.11(2H, dd, CH=CH2), 5.73 to 5.83(2H, m, CH=CH2), 6.41(2H, t, NH), 7.69(2H, S, NHNH), 9.39(2H, S, NHNH)
The solvent solubility of the obtained compound of formula (B) was evaluated. Table 1 shows the results. Upon adding a 3% sodium hypochlorite aqueous solution to the powdery compound of formula (B), foaming and degradation of the compound were observed.

An amount of 0.3 g of the compound of formula (B), 0.1 g of poly(methylhydrosiloxane), and 4.0 mg of a platinum catalyst (trade name: CAT-PL-50T, available from Shin-Etsu Chemical Co., Ltd.) were added to 40 g of dimethyl sulfoxide to obtain a thermosetting composition solution. The solution was applied onto a glass substrate to give a cured film thickness of 0.1 µm, and then dried and cured at 150°C for 10 minutes to obtain a degradable cross-linked product. The degradable cross-linked product was insoluble in water, but when it was immersed in a 3% sodium hypochlorite aqueous solution, foaming and removal of the product from the substrate were observed.

### (Example 2)

An amount of 22.2 g of dimethyl sulfoxide and 1.00 g of 4,4'-hexyl disemicarbazide were mixed in a 50 mL round-bottom flask at 40°C, and 1.89 g of N-(allyloxycarbonyloxy)succinimide was added dropwise to the mixture, followed by stirring at room temperature for four hours. The resulting reaction liquid was added dropwise to 300 g of acetonitrile to obtain a white solid. The white solid was washed with acetonitrile and dried in a vacuum dryer at room temperature to obtain a compound of formula (C) at a yield of 89%.

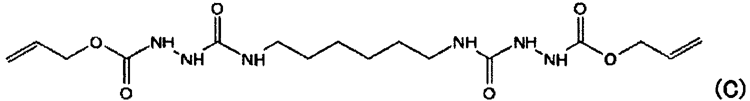

1H-NMR (DMSO, δ ppm) 1.22(4H, S, C6H12), 1.36(4H, t, C6H12), 2.95 to 3.00(4H, m, C6H12), 4.49 to 4.51(4H, m, CH2), 5.19(2H, d, CH=CH2), 5.28 to 5.33(2H, m, CH=CH2), 5.85 to 5.94(2H, m, CH=CH2), 6.30(2H, s, NH), 7.65(2H, S, NHNH), 8.83(2H, S, NHNH)
The solvent solubility of the obtained compound of formula (C) was evaluated. Table 1 shows the results. Upon adding a 3% sodium hypochlorite aqueous solution to the powdery compound of formula (C), foaming and degradation of the compound were observed.

An amount of 0.3 g of the compound of formula (C), 0.1 g of poly(methylhydrosiloxane), and 4.0 mg of a platinum catalyst (trade name: CAT-PL-50T, available from Shin-Etsu Chemical Co., Ltd.) were added to 40 g of propylene glycol monomethyl ether to obtain a thermosetting composition solution. The solution was applied onto a glass substrate to give a cured film thickness of 0.1 µm, and then dried and cured at 130°C for one minute to obtain a degradable cross-linked product. The degradable cross-linked product was insoluble in water, but when it was immersed in a 3% sodium hypochlorite aqueous solution, foaming and removal of the product from the substrate were observed.

### (Example 3)

An amount of 22.4 g of dimethyl sulfoxide and 1.00 g of sebacic acid dihydrazide were mixed in a 50 mL round-bottom flask at room temperature, and 1.90 g of N-(allyloxycarbonyloxy)succinimide was added dropwise to the mixture, followed by stirring for 20 hours. The resulting reaction liquid was added dropwise to 230 g of ion-exchange water to obtain a white solid. The white solid was further washed with ion-exchange water and dried in a vacuum dryer at 40°C to obtain a compound of formula (D) at a yield of 88%.

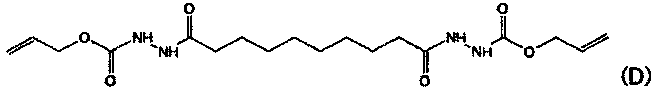

1H-NMR (DMSO, δ ppm) 1.24(8H, S, C8H16), 1.49(4H, t, C8H16), 2.07(4H, t, C8H16), 4.51(4H, d, CH2), 5.19(2H, d, CH=CH2), 5.30(2H, d, CH=CH2), 5.86 to 5.95(2H, m, CH=CH2), 9.04(2H, S, NHNH), 9.58(2H, S, NHNH)
The solvent solubility of the obtained compound of formula (D) was evaluated. Table 1 shows the results. Upon adding a 3% sodium hypochlorite aqueous solution to the powdery compound of formula (D), foaming and degradation of the compound were observed.

The compound of formula (D) and poly(methylhydrosiloxane) were mixed at a weight ratio of 3:1 and dissolved in isopropanol to a concentration of 2% by weight. The resulting solution was diluted with ethyl acetate to a concentration of 1% by weight, and 1 part by weight of a platinum catalyst (trade name: CAT-PL-50T, available from Shin-Etsu Chemical Co., Ltd.) was added thereto to obtain a thermosetting composition solution. The solution was applied onto a glass substrate to give a cured film thickness of 0.1 µm, and then dried and cured at 130°C for one minute to obtain a degradable cross-linked product. The degradable cross-linked product was insoluble in water, but when it was immersed in a 3% sodium hypochlorite aqueous solution, foaming and removal of the product from the substrate were observed.

The compound of formula (D), pentaerythritol tetrakis(3-mercaptobutyrate), and N-methylpyrrolidone were mixed at a weight ratio of 3:2:5 and diluted with a solvent mixture of isopropanol and propylene glycol monomethyl ether (weight ratio 1:1) to a concentration of 15% by weight. Then, 0.2 parts by weight of a photopolymerization initiator (Omnirad 907 available from IGM Resins) and 0.2 parts by weight of a photopolymerization initiator (Omnirad TPO-H available from IGM Resins) were added to the dilution to obtain a photocurable composition solution. The composition was applied onto a glass substrate to give a cured film thickness of 2.5 µm, and then dried at 120°C for three minutes, followed by exposure to ultraviolet rays at 500 mJ/cm² using a UV irradiation device (Unicure UVH-1500M available from USHIO Inc.) to obtain a degradable cross-linked product. The degradable cross-linked product was insoluble in water, but when it was immersed in a 3% sodium hypochlorite aqueous solution, foaming and removal of the product from the substrate were observed.

### (Example 4)

An amount of 10 g of a dual end-type carbinol-modified polydimethylsiloxane having a molecular weight of about 1000 (KF-6000 available from Shin-Etsu Chemical Co., Ltd.), 16 g of acetone, and 6.5 g of triethylamine were mixed in a 100 mL round-bottom flask, and 6.0 g of di(N-succinimidyl) carbonate was added to the mixture, followed by stirring at room temperature overnight. To the reaction liquid were added 47 g of chloroform and 3.1 g of 4-pentenoic acid hydrazide, and they were stirred again at room temperature overnight. The reaction liquid was transferred to a separatory funnel, and 140 g of chloroform was added thereto. Then, the organic layer was washed three times with 140 g of pure water. After the washing, the organic layer was removed by evaporation to obtain a compound having a structure of formula (E) at a yield of 98%. The compound of formula (E) was liquid at 30°C.

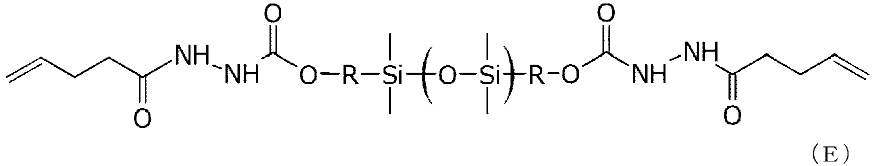

In the formula, R represents a hydrocarbon group.

1H-NMR (CDCl₃, δ ppm) 0.06(76H, br, CH₃), 2.29 to 2.46(8H, m, CH₂CH₂), 4.96 to 5.10(4H, m, CH=CH₂), 5.76 to 5.94(2H, m, CH=CH₂), 7.74(2H, br, NHNH), 8.41(2H, br, NHNH)
The solvent solubility of the obtained compound of formula (E) was evaluated. Table 1 shows the results. Upon adding a 3% sodium hypochlorite aqueous solution to the compound of formula (E), foaming and degradation of the compound were observed.

An amount of 1.61 g of the compound of formula (E), 0.27 g of pentaerythritol tetrakis(mercaptoacetate), 60 mg of a polymerization initiator (Omnirad 907 available from IGM Resins), and 60 mg of a polymerization initiator (Omnirad TPO-H available from IGM Resins) were added to a solvent mixture of 10 g of toluene and 8 g of isopropanol and mixed to obtain a photocurable composition solution. The solution was applied onto a PET film substrate to give a cured film thickness of 0.9 µm,and then dried at 120°C for two minutes, followed by exposure to ultraviolet rays at 500 mJ/cm² using a UV irradiation device (Unicure UVH-1500M available from USHIO Inc.) to obtain a degradable cross-linked product. The degradable cross-linked product was insoluble in ethanol and water, but when it was immersed in a solution prepared by 5-fold dilution of Kitchen Haiter available from Kao Corporation, which contains sodium hypochlorite, with Solmix AP-1 available from Japan Alcohol Trading Co., Ltd., which mainly contains ethanol, foaming and removal of the product from the substrate were observed.

### (Example 5)

An amount of 3.30 g of 1,12-bis(2,5-dioxy-1-pyrrolidinyl) 2,5,8,11-tetraoxadodecanedioate, 26 g of dimethylformamide, and 1.6 g of triethylamine were mixed in a 100 mL round-bottom flask, and 0.88 g of succinic acid dihydrazide was added to the mixture and stirred at room temperature overnight. Next, 0.31 g of 4-hydroxybenzohydrazide was added and further stirred at room temperature for six hours. To the reaction liquid was added 37 g of ethyl acetate, and the mixture was left to stand at -10°C overnight to form a precipitate. The organic solvent was removed by decantation, and the precipitate was washed twice with 35 g of ethyl acetate. Lastly, the solvent was removed by evaporation to obtain a compound having a structure of formula (F) as a white solid at a yield of 79%.

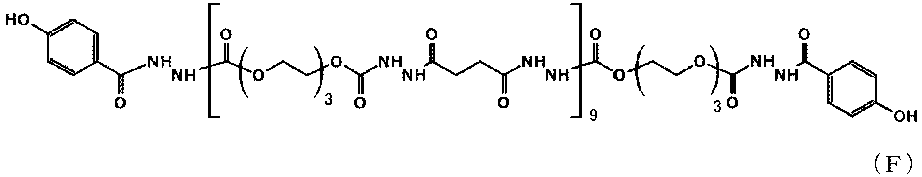

1H-NMR (DMSO, δ ppm) 2.34(36H, br, CH₂CO), 3.54(40H, br, CH₂O), 3.59(40H, br, CH₂O), 4.10(40H, br, CH₂OCO), 6.81(4H, d, benzene ring), 7.72(4H, d, benzene ring), 9.07(20H, br, NHNH), 9.67(20H, br, NHNH), 10.02(2H, br, OH)
The molecular weight was 3,030 as calculated from the proton ratio in 1H-NMR.

The solvent solubility of the obtained compound of formula (F) was evaluated. Table 1 shows the results. Upon adding a 3% sodium hypochlorite aqueous solution to the compound of formula (F), foaming and degradation of the compound were observed.

An amount of 1.0 g of the compound of formula (F), 0.14 g of polyglycerol polyglycidyl ether (GEX-521 available from Nagase ChemteX Corporation), 0.06 g of benzyltriethylammonium chloride, 2 g of methanol, and 0.06 g of a leveling agent (BYK-348 available from BYK Japan KK) were added to 10 g of ion-exchange water to obtain a curable composition solution. The solution was applied onto a glass petri dish to give a cured film thickness of 5 µm, and then dried and cured at 120°C for three hours to obtain a degradable cross-linked product. The degradable cross-linked product was insoluble in ethanol and water, but when it was immersed in a solution prepared by 2-fold dilution of Kitchen Haiter available from Kao Corporation, which contains sodium hypochlorite, with Solmix AP-1 available from Japan Alcohol Trading Co., Ltd., which mainly contains ethanol, foaming and removal of the product from the substrate were observed.

### (Example 6)

An amount of 2.0 g of 4-hydroxybutyric acid hydrazide was dissolved in 170 g of acetonitrile in a 200 mL round-bottom flask at 50°C. To the solution was added 3.4 g of N-(allyloxycarbonyloxy)succinimide, and the mixture was stirred at 40°C for one hour and then further stirred at room temperature overnight. After concentrating the reaction liquid using an evaporator, 30 g of chloroform was added thereto to precipitate N-hydroxysuccinimide as a white solid. The N-hydroxysuccinimide was removed by filtration, and the solvent was removed again by evaporation to obtain a compound of formula (G) at a yield of 90%. The compound of formula (G) was liquid at 50°C.

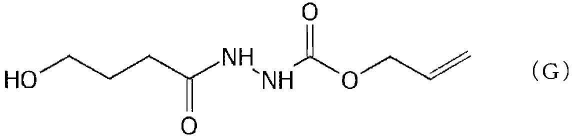

1H-NMR (DMSO, δ ppm) 1.61 to 1.68(2H, m, CH₂), 2.12(2H, t, C=OCH₂), 3.89(2H, q, OCH₂), 4.45(1H, t, OH), 4.51(2H, q, C=OOCH₂), 5.20(1H, d, C=CH2), 5.30(1H, d, C=CH2), 5.85 to 5.96(1H, m, CH=C), 9.04(1H, s, NHNH), 9.59(1H, s, NHNH)
The solvent solubility of the obtained compound of formula (G) was evaluated. Table 1 shows the results. Upon adding a 3% sodium hypochlorite aqueous solution to the compound of formula (G), foaming and degradation of the compound were observed.

An amount of 0.9 g of a nurate-modified product of hexamethylene diisocyanate (BURNOCK DN-901S available from DIC), 1.0 g of the compound of formula (G), and 0.9 g of tetraethylene glycol bis(3-mercaptopropionate) were dissolved in 20 g of a solvent mixture of propylene glycol monomethyl ether and butyl acetate (weight ratio 1:1). To the solution were added 70 mg of a dibutyltin curing catalyst (NEOSTANN U-810 available from Nitto Kasei Co., Ltd.), 40 mg of a photopolymerization initiator (Omnirad 907 available from IGM Resins), and 40 mg of a photopolymerization initiator (Omnirad TPO-H available from IGM Resins) to obtain a curable composition solution. The composition was applied onto a glass plate to give a dried film thickness of 10 µm, and dried and cured at 100°C for 120 minutes, followed by exposure to ultraviolet rays at 500 mJ/cm² using a UV irradiation device (Unicure UVH-1500M available from USHIO Inc.) to obtain a degradable cross-linked product. The degradable cross-linked product was insoluble in water, but when it was immersed in a 3% sodium hypochlorite aqueous solution, foaming and removal of the product from the substrate were observed.

### (Example 7)

An amount of 10.0 g of 2-hydroxyethyl carbazate was added to 38.7 g of glycerol 1,2-carbonate in a 200 mL round-bottom flask, and the mixture was stirred at 80°C for six days. Thereafter, the mixture was returned to room temperature and then added dropwise to 300 g of acetonitrile. The upper layer was removed, and the remaining viscous liquid was dried in a vacuum dryer at 50°C overnight to obtain a compound of formula (H) at a yield of 46%. The compound of formula (H) was liquid at 40°C.

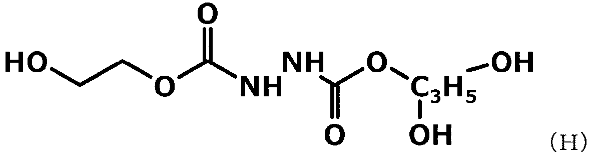

1H-NMR (DMSO, δ ppm) 3.49 to 3.58(2H, m, CH₂), 4.00(2H, t, CH₂), 4.35 to 4.84(7H, m), 9.04(2H, br, NHNH)
The solvent solubility of the obtained compound of formula (H) was evaluated. Table 1 shows the results. Upon adding a 3% sodium hypochlorite aqueous solution to the compound of formula (H), foaming and degradation of the compound were observed.

An amount of 4.0 g of polymethylene polyphenyl polyisocyanate (Millionate MR-200 available from Tosoh Corporation), 2.0 g of the compound of formula (H), 0.2 g of trimethylolpropane, and 50 mg of a dibutyltin curing catalyst (NEOSTANN U-810 available from Nitto Kasei Co., Ltd.) were mixed to obtain a curable composition solution. The composition was applied onto a glass plate to give a dried film thickness of 10 µm, and cured at 70°C for 150 minutes to obtain a degradable cross-linked product. The degradable cross-linked product was insoluble in THF and water, but when it was immersed in a solution prepared by 3-fold dilution of a 3% sodium hypochlorite aqueous solution with THF, foaming and removal of the product from the substrate were observed.

### (Example 8)

An amount of 2.5 g of 3,5-dihydroxybenzoic acid hydrazide was dissolved in 27.3 g of DMSO in a 100 mL round-bottom flask at room temperature. To the solution was added 2.3 g of 2-isocyanatoethyl methacrylate (Karenz MOI available from Resonac), and the mixture was stirred for one day. The resulting reaction liquid was mixed with 300 g of toluene. The upper layer was removed, and 280 g of methyl isobutyl ketone was added to the remaining lower layer to precipitate a solid. The solid was collected by filtration, washed with 150 g of methyl isobutyl ketone, and dried in a vacuum dryer at 30°C overnight to obtain a compound of formula (I) at a yield of 82%.

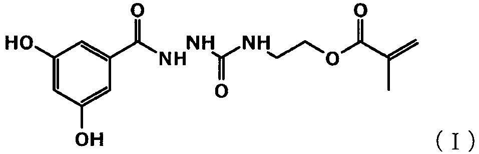

1H-NMR (DMSO, δ ppm) 1.88(3H, s, CH₃), 3.33(2H, q, CH₂), 4.08(2H, t, CH₂), 5.67(1H, br, C=CH₂), 6.07(1H, br, C=CH₂), 6.38(1H, t, benzene ring) 6.55(1H, t, NH) 6.72(2H, d, benzene ring) 7.89(1H, br, NHNH), 9.47(2H, br, OH), 9.88(1H, br, NHNH)
The solvent solubility of the obtained compound of formula (I) was evaluated. Table 2 shows the results. Upon adding a 3% sodium hypochlorite aqueous solution to the compound of formula (I), foaming and degradation of the compound were observed.

An amount of 2.0 g of polyethylene glycol diglycidyl ether (EX-830 available from Nagase ChemteX Corporation), 1.0 g of the compound of formula (I), and 0.06 g of benzyltriethylammonium chloride were mixed with 20 g of methanol. To the mixture were added 4 g of N-methylpyrrolidone and 0.05 g of a photopolymerization initiator (Omnirad 907 available from IGM Resins) to obtain a curable composition solution. The composition was applied onto a glass petri dish to give a dried film thickness of 5 µm, and then dried and cured at 120°C for two minutes, followed by exposure to ultraviolet rays at 500 mJ/cm² using a UV irradiation device (Unicure UVH-1500M available from USHIO Inc.). The product was further cured at 120°C for two hours to obtain a degradable cross-linked product. The degradable cross-linked product was insoluble in isopropanol and water, but when it was immersed in a solution prepared by 2-fold dilution of a 3% sodium hypochlorite aqueous solution with isopropanol, foaming and removal of the product from the substrate were observed.

### (Example 9)

An amount of 2.0 g of 3-mercaptopropane hydrazide was dissolved in 26 g of THF in a 100 mL round-bottom flask, and 3.0 g of poly(hexamethylene diisocyanate) (available from Sigma Aldrich) was added dropwise to the solution, followed by stirring at room temperature overnight to precipitate a white solid. The white solid was collected by decantation and dried in a vacuum dryer to obtain a cross-linking agent having a structure of formula (J) at a yield of 94%.

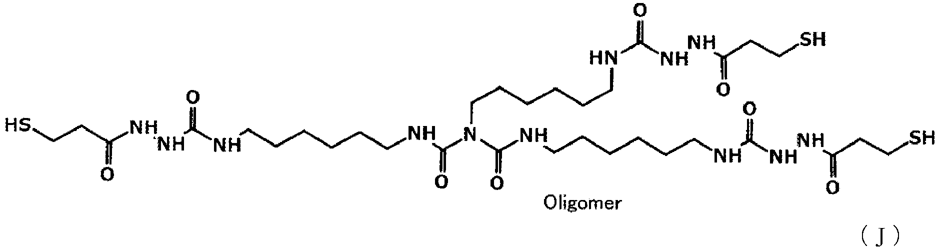

1H-NMR (DMSO, δ ppm) 1.24 to 1.50(24H, m, CH₂), 2.41(6H, t, C=OCH₂), 2.67(6H, t, SCH₂), 2.97 to 3.10(12H, m, NCH₂), 6.26(3H, s, NH), 7.67(3H, s, NH), 8.21(2H, br, NH), 9.50(3H, s, NH)
The solvent solubility of the obtained compound of formula (J) was evaluated. Table 2 shows the results. Upon adding a 3% sodium hypochlorite aqueous solution to the compound of formula (J), foaming and degradation of the compound were observed.

An amount of 1.2 g of the compound of formula (J) and 2.3 g of a reactive silicone oil having an acrylate group at both terminals (X-22-2445 available from Shin-Etsu Silicone) were dissolved in a solvent mixture of 20 g of N-methylpyrrolidone, 4 g of toluene, and 10 g of methyl ethyl ketone. To the solution were added 0.3 g of a leveling agent (BYK-307 available from BYK Japan KK), 0.2 g of a photopolymerization initiator (Omnirad 907 available from IGM Resins), and 0.2 g of a photopolymerization initiator (Omnirad TPO-H available from IGM Resins) to obtain a curable composition solution. The composition was applied onto a PET film to give a dried film thickness of 0.8 µm, and then dried at 120°C for 10 minutes, followed by curing using ultraviolet rays at 2000 mJ/cm² to obtain a degradable cross-linked product. The degradable cross-linked product was insoluble in water, but when it was immersed in a 3% sodium hypochlorite aqueous solution, foaming and removal of the product from the substrate were observed.

### (Example 10)

An amount of 10 g of polymethyl acrylate with a molecular weight of about 1000 having a hydroxy group at one terminal (UMM-1001 available from Soken Chemical & Engineering Co., Ltd.), 13 g of acetonitrile, and 5.1 g of triethylamine were mixed in a 100 mL round-bottom flask, and 4.7 g of di(N-succinimidyl) carbonate was added to the mixture, followed by stirring at room temperature overnight. Next, 1.4 g of trimesic acid trihydrazide and 80 g of dimethyl sulfoxide were added to the mixture, followed by stirring at 60°C for four hours. The reaction liquid was transferred to a separatory funnel, and 210 g of ethyl acetate and 210 g of pure water were added thereto, followed by removing the aqueous layer. Further, the organic layer was washed four times with 90 g of pure water, and the organic solvent was removed by evaporation. An amount of 4 g of the residue was collected and dissolved in 30 g of acetonitrile, and then 10 g of ethanol was added, followed by concentration using an evaporator. This process was performed twice. The purified residue was transferred to a 500 mL round-bottom flask and dissolved in 168 g of ethanol and 100 g of acetonitrile, and 38 g of hydrazine monohydrate was introduced in four portions into the solution, followed by stirring at room temperature for four days. The reaction liquid was added dropwise to 140 g of ice-cooled isopropanol to precipitate a solid.

The obtained solid was washed with 40 g of acetonitrile and then dried in a vacuum dryer at 50°C for three hours. An amount of 1.0 g of the dried solid was transferred to a 30 mL round-bottom flask and dissolved in 11.1 g of DMSO, and 2.3 g of N-(allyloxycarbonyloxy)succinimide was added to the solution, followed by stirring at room temperature overnight. The reaction liquid was added dropwise to 87 g of ion-exchange water to precipitate a solid. The obtained solid was dissolved in 6 g of ethanol and then added dropwise to 60 g of methyl isobutyl ketone to precipitate a solid again. The obtained solid was dried in a vacuum dryer at 30°C overnight to obtain 0.5 g of a compound of formula (K).

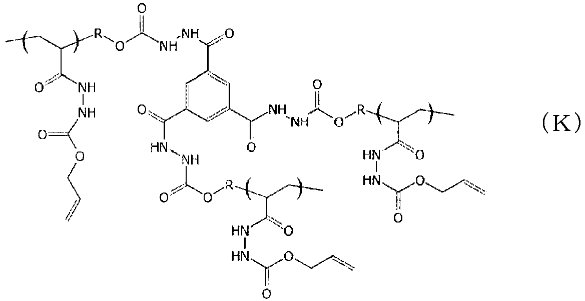

In the formula, R represents a hydrocarbon group.

1H-NMR (DMSO, δ ppm) 1.4 to 1.9(75H, m, CH₂), 1.95 to 2.41(37H, m, CH), 4.55(71H, d, OCH₂), 5.21(35H, t, C=CH₂), 5.32(35H, d, C=CH₂), 5.91(35H, br, C=CH), 8.48(3H, s, benzene ring) 9.30(72H, br, NHNH), 10.61(3H, br, NHNH)
The solvent solubility of the obtained compound of formula (K) was evaluated. Table 2 shows the results. Upon adding a 3% sodium hypochlorite aqueous solution to the compound of formula (K), foaming and degradation of the compound were observed.

The compound of formula (K), pentaerythritol tetrakis(mercaptoacetate), and the compound of formula (I) were mixed at a weight ratio of 3:2:2 and diluted with methanol to a concentration of 15% by weight. Then, 0.2 parts by weight of a photopolymerization initiator (Omnirad 907 available from IGM Resins) and 0.2 parts by weight of a photopolymerization initiator (Omnirad TPO-H available from IGM Resins) were added to the dilution to obtain a photocurable composition solution. The composition was applied onto a glass substrate to give a cured film thickness of 4 µm, and then dried at 120°C for one minute, followed by exposure to ultraviolet rays at 500 mJ/cm² using a UV irradiation device (Unicure UVH-1500M available from USHIO Inc.) to obtain a degradable cross-linked product. The degradable cross-linked product was insoluble in water, but when it was immersed in a 3% sodium hypochlorite aqueous solution, foaming and removal of the product from the substrate were observed.

### (Example 11)

An amount of 5.0 g of succinic acid dihydrazide was dissolved in 30 g of methyl sulfoxide in a 100 mL three-necked flask, and 15.7 g of diethyl 2-isocyanatoglutarate was added to the solution, followed by stirring at room temperature for one day. Then, 24.7 g of hydrazine monohydrate was added, and the mixture was further stirred for one day. The reaction liquid was added dropwise to 900 g of methanol to precipitate a solid. The obtained solid was washed with 300 g of methanol and dried in a vacuum dryer at 40°C for three hours. An amount of 5 g of the dried solid was transferred to a 100 mL round-bottom flask and dissolved in 23 g of DMSO, and 7.3 g of N-(allyloxycarbonyloxy)succinimide was added to the solution, followed by stirring at room temperature for one day. The reaction liquid was added dropwise to 550 g of acetonitrile to precipitate a solid. The solid was washed with 250 g of acetonitrile and dried in a vacuum dryer at 50°C for three hours to obtain 2.6 g of a compound of formula (L).

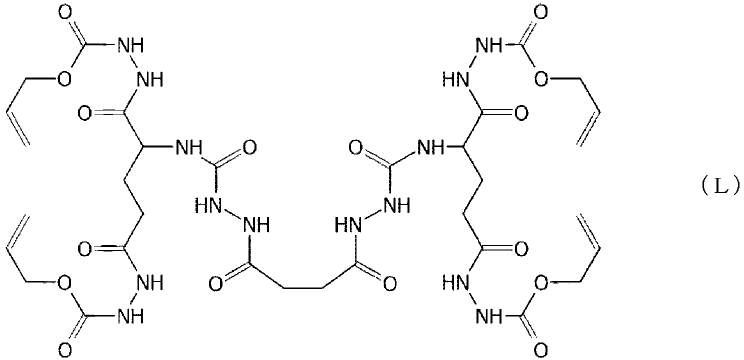

1H-NMR (DMSO, δ ppm) 1.68 to 1.91(4H, m, CH₂), 2.04 to 2.17(4H, m, C=OCH₂), 2.37(4H, br, C=OCH₂), 4.07 to 4.13 (2H, m, C=OCH), 4.51(8H, br, OCH₂), 5.19(4H, d, C=CH₂), 5.30(4H, d, C=CH₂), 5.89(4H, br, CH=C), 6.52(2H, br, NC=ONHC), 7.90(2H, s, NHNH), 9.03(2H, br, NHNH), 9.19(2H, br, NHNH), 9.57(2H, br, NHNH), 9.64(2H, br, NHNH), 9.81(2H, br, NHNH)
The solvent solubility of the obtained compound of formula (L) was evaluated. Table 2 shows the results. Upon adding a 3% sodium hypochlorite aqueous solution to the compound of formula (L), foaming and degradation of the compound were observed.

An amount of 0.26 g of the compound of formula (L) and 0.22g of tetraethylene glycol bis(3-mercaptopropionate) were dissolved in 4 g of a solvent mixture of methanol and N-methylpyrrolidone (weight ratio 3:1). To the solution were added 8 mg of a photopolymerization initiator (Omnirad 907 available from IGM Resins) and 8 mg of a photopolymerization initiator (Omnirad TPO available from IGM Resins) to obtain a curable composition. The composition was applied onto a glass plate to give a dried film thickness of 2 µm, and then exposed to ultraviolet rays at 500 mJ/cm² using a UV irradiation device (Unicure UVH-1500M available from USHIO Inc.) to obtain a degradable cross-linked product. The degradable cross-linked product was insoluble in water, but when it was immersed in a 3% sodium hypochlorite aqueous solution, foaming and removal of the product from the substrate were observed.

### (Example 12)

An amount of 14.5 g of N-hydroxysuccinimide was dissolved in 377 g of THF in a 1 L round-bottom flask, and 9.5 g of isophthalic acid and 24.3 g of 1-ethyl-3-(3-dimethylaminopropyl)carbodiimide hydrochloride were added to the solution, followed by stirring at room temperature overnight. After the resulting mixture was concentrated using an evaporator, 310 g of chloroform was added, and the mixture was transferred to a separatory funnel. The organic layer was washed four times with 200 g of ion-exchange water. The organic layer was dried over 5.8 g of magnesium sulfate and then concentrated using an evaporator. The concentrated liquid was added dropwise to 150 g of isopropanol to precipitate a solid. The obtained solid was dried in a vacuum dryer at 25°C overnight. An amount of 13.6 g of 6-hydroxyhexanoic acid hydrazide, 18 g of triethylamine, and 40 g of DMF were mixed in a 100 mL round-bottom flask, and 15.3 g of the dried solid was added thereto. After stirring at room temperature overnight, the reaction liquid was added dropwise to 800 g of acetonitrile to precipitate a white powder. The white powder was collected by filtration, washed with 200 g of acetonitrile, and then dried in a vacuum dryer at 50°C for three hours. An amount of 6 g of the dried white powder was transferred to a 100 mL round-bottom flask and dissolved in 36 g of DMF, and then 8.7 g of triethylamine and 7.3 g of di(N-succinimidyl) carbonate were added to the solution, followed by stirring at room temperature for 90 minutes. Thereafter, 3.4 g of 2-hydroxyethyl carbazate was added, and the mixture was further stirred overnight. The reaction liquid was added dropwise to 800 g of acetonitrile to precipitate a solid. The solid was washed with 200 g of acetonitrile and dried in a vacuum dryer at 50°C for three hours to obtain 6.7 g of a compound of formula (M).

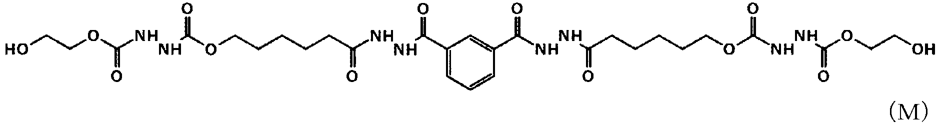

1H-NMR (DMSO, δ ppm) 1.31 to 1.63(12H, m, CH₂), 2.17 to 2.24(4H, m, O=CCH₂), 3.55(4H, q, OCH₂), 3.99 to 4.023(8H, q, O=COCH₂), 4.77(2H, br, OH), 7.62(1H, t, benzene ring), 8.03(2H, dd, benzene ring), 8.37(1H, s, benzene ring), 9.00(2H, br, NHNH), 9.03(2H, br, NHNH), 9.99(2H, br, NHNH), 10.40(2H, br, NHNH)
The solvent solubility of the obtained compound of formula (M) was evaluated. Table 2 shows the results. Upon adding a 3% sodium hypochlorite aqueous solution to the compound of formula (M), foaming and degradation of the compound were observed.

An amount of 1.5 g of polymethylene polyphenyl polyisocyanate (Millionate MR-200 available from Tosoh Corporation), 4.0 g of the compound of formula (M), 50 mg of a dibutyltin curing catalyst (NEOSTANN U-810 available from Nitto Kasei Co., Ltd.), and 5 g N-methylpyrrolidone were mixed to obtain a curable composition solution. The composition was applied onto a glass plate to give a dried film thickness of 2 µm, and then dried and cured at 120°C for 150 minutes to obtain a degradable cross-linked product. The degradable cross-linked product was insoluble in THF and water, but when it was immersed in a solution prepared by 3-fold dilution of a 3% sodium hypochlorite aqueous solution with THF, foaming and removal of the product from the substrate were observed.

### (Example 13)

An amount of 10 g of a triol polypropylene glycol with a molecular weight of about 1500 (available from FUJIFILM Wako Pure Chemical Corporation), 40 g of acetonitrile, and 18.2 g of triethylamine were mixed in a 100 mL round-bottom flask, and 15.3 g of di(N-succinimidyl) carbonate was added to the mixture, followed by stirring at room temperature overnight. The precipitated solid was removed by filtration, and then 54 g of chloroform and 100 g of pure water were added to the filtrate to perform a liquid separation process. After removing the aqueous layer, the solvent was removed by evaporation to a concentration of 80%, and 11 g of acetonitrile and 2.8 g of triethylamine were added thereto. Then, 0.22 g of 2-hydroxyethyl carbazate was added, and the mixture was stirred at room temperature for four hours. The completion of the reaction was confirmed by NMR. Subsequently, 1.9 g of α-resorcylic acid hydrazide was added, and the mixture was stirred at room temperature for four hours. After concentrating the reaction liquid using an evaporator, 103 g of chloroform was added, and the mixture was transferred again to a separatory funnel. The organic layer was washed twice with 70 g of ion-exchange water. Then, the solvent was removed by evaporation using an evaporator to obtain a degradable compound having a structure of formula (N) at a yield of 61%.

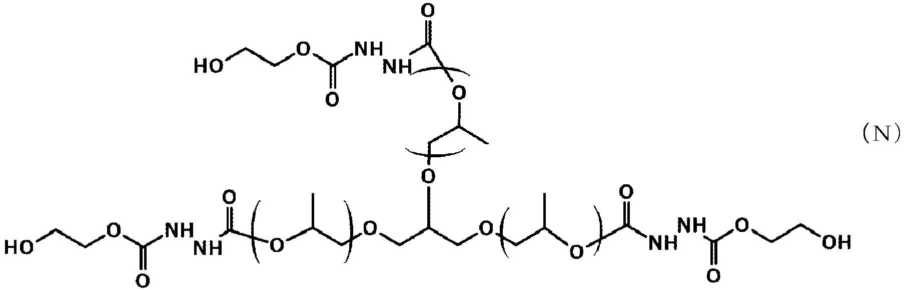

1H-NMR (DMSO, δ ppm) 1.05(77H, d, CH₃), 3.32 to 3.67(80H, m, CH, CH₂), 4.00(6H, t, O=COCH₂), 4.75(3H, t, OH), 8.97(3H, br, NHNH), 8.99(3H, br, NHNH)
The solvent solubility of the obtained compound of formula (N) was evaluated. Table 2 shows the results. Upon adding a 3% sodium hypochlorite aqueous solution to the compound of formula (N), foaming and degradation of the compound were observed.

An amount of 1.0 g of polymethylene polyphenyl polyisocyanate (Millionate MR-200 available from Tosoh Corporation), 5.0 g of the compound of formula (N), 60 mg of a dibutyltin curing catalyst (NEOSTANN U-810 available from Nitto Kasei Co., Ltd.), and 0.5 g of methyl isobutyl ketone were mixed to obtain a curable composition solution. The composition was applied onto a polyester non-woven fabric to give a dried film thickness of 200 µm, and then heated at 60°C for 10 minutes, followed by heating at 40°C for six hours to obtain a degradable cross-linked product. The degradable cross-linked product was insoluble in isopropanol and water, but when it was immersed in a solution prepared by 2-fold dilution of a 3 wt% sodium dichloroisocyanurate aqueous solution with isopropanol, foaming and removal of the product from the substrate were observed.

### (Solvent solubility evaluation)

An amount of 0.5 mL of the solvent indicated in Table 1 or 2 was added to 2 mg of the degradable cross-linking agent of each of Examples 1 to 13 and Comparative Example 1, and the mixture was stirred. The degree of dissolution was visually observed. When an undissolved portion remained at room temperature, the mixture was sufficiently heated with a heat gun and then visually observed again. The scoring was as follows: 3 points for those that completely dissolved at room temperature; 2 points for those in which an undissolved portion remained at room temperature but completely dissolved after heating; 1 point for those in which a slight amount of an undissolved portion remained after heating; and 0 points for those in which a large amount of an undissolved portion remained after heating. Here, the degradable cross-linking agents in liquid form were evaluated for compatibility with the solvents. Tables 1 and 2 show the results.

**[Table 1]**

| | | Comparative Example 1 | Example 1 | Example 2 | Example 3 | Example 4 | Example 5 | Example 6 | Example 7 |
|---|---|---|---|---|---|---|---|---|---|
| Degradable cross-linking agent | | **Formula** (A) | Formula (B) | Formula (C) | Formula (D) | Formula (E) | Formula (F) | Formula (G) | Formula (H) |
| Solvent type | Isopropanol | 0 | 1 | 1 | 3 | 3 | 0 | 3 | 1 |
| | Methanol | 0 | 2 | 2 | 3 | 3 | 1 | 3 | 3 |
| | Propylene glycol monomethyl ether | 0 | 0 | 2 | 3 | 3 | 0 | 3 | 1 |
| | Methyl isobutyl ketone | 0 | 0 | 0 | 2 | 3 | 0 | 2 | 0 |
| | N-methylpyrrolidone | 0 | 3 | 3 | 3 | 0 | 3 | 3 | 3 |

**[Table 2]**

| | | Example 8 | Example 9 | Example 10 | Example 11 | Example 12 | Example 13 |
|---|---|---|---|---|---|---|---|
| Degradable cross-linking agent | | Formula (I) | Formula (J) | Formula (K) | Formula (L) | Formula (M) | Formula (N) |
| Solvent type | Isopropanol | 0 | 1 | 0 | 1 | 0 | 3 |
| | Methanol | 3 | 1 | 3 | 2 | 3 | 3 |
| | Propylene glycol monomethyl ether | 0 | 0 | 1 | 3 | 3 | 3 |
| | Methyl isobutyl ketone | 0 | 0 | 0 | 1 | 0 | 3 |
| | N-methylpyrrolidone | 3 | 1 | 3 | 3 | 3 | 3 |

The compound of formula (A) of Comparative Example 1 was poorly soluble in all the solvents. The compounds of Examples 1 to 13 were soluble in a plurality of the solvents. In particular, the compounds of Examples 1 to 7 and Examples 10 to 13, having a -NHNHCOO- structure, exhibited high solubility. The compound of Example 9, having a branched structure, was slightly less soluble than the compound of Example 3 but exhibited an acceptable level of solubility. The compounds of Example 5 and Example 10, having a high molecular weight, were slightly less soluble than the compound of Example 3 but exhibited an acceptable level of solubility. The compounds of Examples 7 and 8, with localized reactive functional groups, were slightly less soluble than the compound of Example 3 but exhibited an acceptable level of solubility.

Embodiment 1 of the present disclosure relates to a degradable cross-linking agent, including a compound represented by the following formula (1): 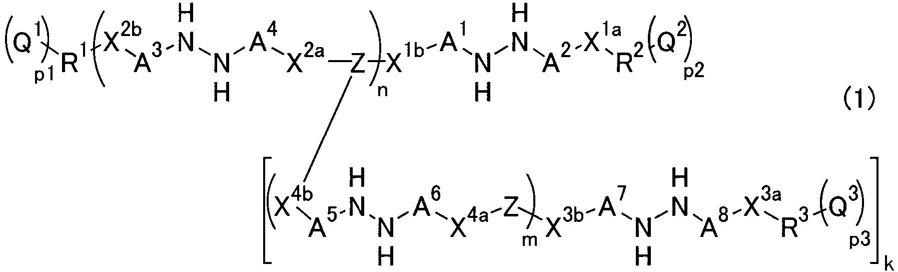 wherein
- n ≥ 0, k ≥ 0, m ≥ 0, p1 ≥ 1, p2 ≥ 1, and p3 ≥ 1;
- R¹, R², and R³ each independently represent a single bond or a C1-C500 hydrocarbon optionally containing a substituent or a heteroatom;
- Q¹, Q², and Q³ each independently represent at least one reactive functional group selected from the group consisting of a hydroxy group, an amino group, a thiol group, a hydrazide group, a carboxylic acid group, an acid anhydride group, a vinyl group, an allyl group, an acrylate group, a methacrylate group, a crotonate group, an isoprenyl group, an acrylamide group, a methacrylamide group, a crotonamide group, an epoxy group, an oxetane group, an oxazoline group, an isocyanate group, a carbodiimide group, a methylol group, a silanol group, a hydroxysilyl group, and an alkoxysilyl group;
- X^{1a}, X^{1b}, X^{2a}, X^{2b}, X^{3a}, X^{3b}, X^{4a}, and X^{4b} each independently represent an oxygen atom, a sulfur atom, a single bond, a C1-C20 hydrocarbon group optionally containing a substituent, or -NB- where B represents a hydrogen atom or a hydrocarbon group, and at least one of X^{1a}, X^{1b}, X^{2a}, X^{2b}, X^{3a}, X^{3b}, X^{4a}, and X^{4b} is an oxygen atom, a sulfur atom, or -NB- where B represents a hydrogen atom or a hydrocarbon group;
- A¹ to A⁸ each represent a carbonyl group or a single bond, at least one of A¹ and A² is a carbonyl group, at least one of A³ and A⁴ is a carbonyl group, at least one of A⁵ and A⁶ is a carbonyl group, and at least one of A⁷ and A⁸ is a carbonyl group; and
- each Z independently represents a divalent or higher-valent group containing a siloxane structure or a C1-C500 hydrocarbon group optionally containing a heteroatom.

Embodiment 2 of the present disclosure is the degradable cross-linking agent according to Embodiment 1,
wherein p1 ≥ 2, p2 ≥ 2, or p3 ≥ 2, and
the plurality of reactive functional groups as Q¹ are different from each other, or the plurality of reactive functional groups as Q² are different from each other, or the plurality of reactive functional groups as Q³ are different from each other.

Embodiment 3 of the present disclosure is the degradable cross-linking agent according to Embodiment 1,
wherein p1 = 1, p2 = 1, or p3 = 1.

Embodiment 4 of the present disclosure is the degradable cross-linking agent according to any one of Embodiments 1 to 3, which has at least one degradable moiety represented by the following formula (2):

-X⁵-CO¹-NH-NH-CO²-X⁶- (2)

wherein X⁵ and X⁶ are different from each other and each represent an oxygen atom, a sulfur atom, a single bond, a C1-C20 hydrocarbon group optionally containing a substituent, or -NB- where B represents a hydrogen atom or a hydrocarbon group; and C¹ and C² each represent a carbonyl group or a single bond, and at least one of C¹ and C² is a carbonyl group.

Embodiment 5 of the present disclosure is the degradable cross-linking agent according to any one of Embodiments 1 to 4,
wherein X^{1a} or X^{1b} is an oxygen atom.

Embodiment 6 of the present disclosure is the degradable cross-linking agent according to any one of Embodiments 1 to 5,
wherein n ≥ 1.

Embodiment 7 of the present disclosure is the degradable cross-linking agent according to any one of Embodiments 1 to 6,
wherein n ≥ 1,
at least one of X^{1a}, X^{1b}, X^{2a}, X^{2b}, X^{3a}, X^{3b}, X^{4a}, and X^{4b} is an oxygen atom,
at least one of X^{1a}, X^{1b}, X^{2a}, X^{2b}, X^{3a}, X^{3b}, X^{4a}, and X^{4b} is a single bond or a C1-C20 hydrocarbon group optionally containing a substituent, and
at least one of X^{1a}, X^{1b}, X^{2a}, X^{2b}, X^{3a}, X^{3b}, X^{4a}, and X^{4b} is -NB- where B represents a hydrogen atom or a hydrocarbon group.

Embodiment 8 of the present disclosure is the degradable cross-linking agent according to any one of Embodiments 1 to 7, which has a molecular weight of 2000 or less.

Embodiment 9 of the present disclosure is the degradable cross-linking agent according to any one of Embodiments 1 to 8,
wherein k ≥ 1.

Embodiment 10 of the present disclosure relates to a composition, containing:
the degradable cross-linking agent according to any one of Embodiments 1 to 9, and
at least one selected from the group consisting of a curable resin, a polymerization initiator, and a solvent.

Embodiment 11 of the present disclosure is the composition according to Embodiment 10, which contains substantially no solvent with a boiling point of higher than 150°C.

Embodiment 12 of the present disclosure relates to a degradable cross-linked product, formed from the composition according to Embodiment 10 or 11.

Embodiment 13 of the present disclosure is the degradable cross-linked product according to Embodiment 12, which is degradable with an oxidizing agent.

Embodiment 14 of the present disclosure relates to a method for degrading a degradable cross-linked product, including
bringing the degradable cross-linked product according to Embodiment 12 or 13 into contact with an aqueous solution containing an oxidizing agent at 100°C or lower.

## Claims

1. A degradable cross-linking agent, comprising a compound represented by the following formula (1): 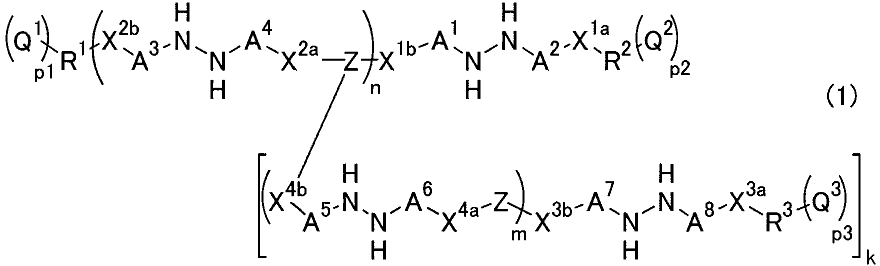 wherein
- n ≥ 0, k ≥ 0, m ≥ 0, p1 ≥ 1, p2 ≥ 1, and p3 ≥ 1;
- R¹, R², and R³ each independently represent a single bond or a C1-C500 hydrocarbon optionally containing a substituent or a heteroatom;
- Q¹, Q², and Q³ each independently represent at least one reactive functional group selected from the group consisting of a hydroxy group, an amino group, a thiol group, a hydrazide group, a carboxylic acid group, an acid anhydride group, a vinyl group, an allyl group, an acrylate group, a methacrylate group, a crotonate group, an isoprenyl group, an acrylamide group, a methacrylamide group, a crotonamide group, an epoxy group, an oxetane group, an oxazoline group, an isocyanate group, a carbodiimide group, a methylol group, a silanol group, a hydroxysilyl group, and an alkoxysilyl group;
- X^{1a}, X^{1b}, X^{2a}, X^{2b}, X^{3a}, X^{3b}, X^{4a}, and X^{4b} each independently represent an oxygen atom, a sulfur atom, a single bond, a C1-C20 hydrocarbon group optionally containing a substituent, or -NB- where B represents a hydrogen atom or a hydrocarbon group, and at least one of X^{1a}, X^{1b}, X^{2a}, X^{2b}, X^{3a}, X^{3b}, X^{4a}, and X^{4b} is an oxygen atom, a sulfur atom, or -NB- where B represents a hydrogen atom or a hydrocarbon group;
- A¹ to A⁸ each represent a carbonyl group or a single bond, at least one of A¹ and A² is a carbonyl group, at least one of A³ and A⁴ is a carbonyl group, at least one of A⁵ and A⁶ is a carbonyl group, and at least one of A⁷ and A⁸ is a carbonyl group; and
- each Z independently represents a divalent or higher-valent group containing a siloxane structure or a C1-C500 hydrocarbon group optionally containing a heteroatom.

2. The degradable cross-linking agent according to claim 1,
wherein p1 ≥ 2, p2 ≥ 2, or p3 ≥ 2, and
the plurality of reactive functional groups as Q¹ are different from each other, or the plurality of reactive functional groups as Q² are different from each other, or the plurality of reactive functional groups as Q³ are different from each other.

3. The degradable cross-linking agent according to claim 1 or 2,
wherein p1 = 1, p2 = 1, or p3 = 1.

4. The degradable cross-linking agent according to any one of claims 1 to 3, which has at least one degradable moiety represented by the following formula (2):
-X⁵-C^{l}-NH-NH-C²-X⁶- (2)
wherein X⁵ and X⁶ are different from each other and each represent an oxygen atom, a sulfur atom, a single bond, a C1-C20 hydrocarbon group optionally containing a substituent, or -NB- where B represents a hydrogen atom or a hydrocarbon group; and C¹ and C² each represent a carbonyl group or a single bond, and at least one of C¹ and C² is a carbonyl group.

5. The degradable cross-linking agent according to any one of claims 1 to 4,
wherein X^{1a} or X^{1b} is an oxygen atom.

6. The degradable cross-linking agent according to any one of claims 1 to 5,
wherein n ≥ 1.

7. The degradable cross-linking agent according to any one of claims 1 to 6,
wherein n ≥ 1,
at least one of X^{1a}, X^{1b}, X^{2a}, X^{2b}, X^{3a}, X^{3b}, X^{4a}, and X^{4b} is an oxygen atom,
at least one of X^{1a}, X^{1b}, X^{2a}, X^{2b}, X^{3a}, X^{3b}, X^{4a}, and X^{4b} is a single bond or a C1-C20 hydrocarbon group optionally containing a substituent, and
at least one of X^{1a}, X^{1b}, X^{2a}, X^{2b}, X^{3a}, X^{3b}, X^{4a}, and X^{4b} is -NB- where B represents a hydrogen atom or a hydrocarbon group.

8. The degradable cross-linking agent according to any one of claims 1 to 7, which has a molecular weight of 2000 or less.

9. The degradable cross-linking agent according to any one of claims 1 to 8,
wherein k ≥ 1.

10. A composition, comprising:
the degradable cross-linking agent according to any one of claims 1 to 9, and
at least one selected from the group consisting of a curable resin, a polymerization initiator, and a solvent.

11. The composition according to claim 10, which comprises substantially no solvent with a boiling point of higher than 150°C.

12. A degradable cross-linked product, formed from the composition according to claim 10.

13. The degradable cross-linked product according to claim 12, which is degradable with an oxidizing agent.

14. A method for degrading a degradable cross-linked product, comprising
bringing the degradable cross-linked product according to claim 12 or 13 into contact with an aqueous solution containing an oxidizing agent at 100°C or lower.
